# EUROPEAN PATENT APPLICATION

(11) **EP 4 614 157 A1**
(43) Date of publication of application: **10.09.2025**
(21) Application number: 23885502.7
(22) Date of filing: 13.10.2023
(51) Int. Cl.: G01N 35/02, G01N 33/543, G01N 33/553, B03C 1/00, B03C 1/01, B03C 1/24, B03C 1/28

(54) **MAGNETIC COLLECTING UNIT AND TEST DEVICE**

(30) Priority: 31.10.2022 JP 2022175124
(71) Applicant: FUJIFILM Corporation, Tokyo 106-8620 (JP)
(72) Inventor: HIBE, Daisuke, Ashigarakami-gun, Kanagawa 258-8538 (JP)
(74) Representative: Dehns Germany Partnerschaft mbB
(86) International application number: PCT/JP2023/037294
(87) International publication number: WO 2024/095740

(57) **Abstract**

A magnetic collection unit is a magnetic collection unit which, during a washing treatment of separating a labeling substance bound to an examination target substance and a labeling substance not bound to the examination target substance in an examination device using magnetic particles in a solid phase in an antigen-antibody reaction, generates a magnetic field in an inside of a reaction cell accommodating a suspension containing the magnetic particles and magnetically collects the magnetic particles in the suspension on an inner wall surface of the reaction cell, the magnetic collection unit including a magnetic field generation unit that includes a magnet having a length at least equal to or longer than a distance from a liquid surface of the suspension in the reaction cell to a bottom surface of the reaction cell and generating a magnetic field across a range from the liquid surface to the bottom surface; and a moving mechanism that moves the magnetic field generation unit in a depth direction from the liquid surface toward the bottom surface in a state in which an upper end of the magnet is positioned at the liquid surface or above the liquid surface.

## Description

### BACKGROUND OF THE INVENTION

### 1. Field of the Invention

The present disclosure relates to a magnetic collection unit and an examination device.

### 2. Description of the Related Art

An examination device that quantitatively or qualitatively detects an examination target substance in a specimen has been known. Many of such examination devices use an immunoassay principle, and examples thereof include a chemiluminescent enzyme immunological analysis device and a fluorescence immunological analysis device (for example, JP2016-085093A).

Such an examination device carries out a detection treatment of detecting an examination target substance in a specimen by detecting luminescence or fluorescence based on a labeling substance such as an enzyme label or a fluorescent label attached to the examination target substance in the specimen by using an immunoreaction. In addition, before such a detection treatment of the examination target substance, a pretreatment such as attaching a labeling substance to the examination target substance in the specimen is carried out on the specimen. In some cases, the examination device is configured to automatically execute the pretreatment and the detection treatment and output the detection result in a case where the pretreatment and the detection treatment are automated and a specimen collection container accommodating the collected specimen is loaded.

In such an automated examination device, in order to attach a labeling substance to an examination target substance in a specimen, for example, such a treatment as described later is carried out using magnetic particles as a solid phase. First, in a reaction cell, magnetic particles modified with a first binding substance (for example, a primary antibody) that specifically binds to the target substance (for example, an antigen) are mixed with the specimen, and thus the target substance and the first binding substance are bound to each other to generate an immune complex. As a result, the target substance is captured by the magnetic particles through the first binding substance. Thereafter, the generated immune complex is separated from the immune complex and a component derived from the specimen (unreacted substance) which does not form the immune complex, that is, so-called bound/free (B/F) separation is carried out. During the B/F separation, the liquid is suctioned in a state in which the magnetic particles are temporarily adsorbed to an inner wall surface of the reaction cell by a magnet disposed outside the reaction cell. Thereafter, a washing solution is discharged to the reaction cell, and the mixed liquid is suctioned and discharged in a state in which the washing solution and the magnetic particles are mixed, whereby the magnetic particles are washed. Next, a labeling reagent containing a second binding substance (for example, a secondary antibody) that specifically binds to the target substance is mixed with the magnetic particles, where the second binding substance has been bound to the labeling substance. As a result, the target substance captured on the magnetic particles through the first binding substance and the second binding substance is bound to each other, and thus a sandwich type immune complex in which the target substance is sandwiched between the first binding substance and the second binding substance is generated. Thereafter, the magnetic particles are washed again by mixing the washing solution and the magnetic particles for the B/F separation. In a case where the label is an enzyme label, the magnetic particles and a reagent containing a luminescent substrate are further mixed and subjected to the detection treatment.

JP2006-218442A proposes a magnetic collection device (magnetic collection unit) that obtains a high gradient magnetic field, where the magnetic collection device is for shortening the time required for adsorbing magnetic particles to an inner wall surface during the B/F separation. The magnetic collection device according to JP2006-218442A is a pair of magnets that are obtained by disposing the same poles repelling each other to face each other, where a separation container (reaction cell) is disposed to be brought close to a gap between the magnetic poles to increase the magnetic field strength that is generated in a reaction cell.

### SUMMARY OF THE INVENTION

By providing the pair of magnets described in JP2006-218442A, it is possible to magnetically collect the magnetic particles on the inner surface of the side wall surface of the reaction cell that faces the gap between the pair of magnetic poles in a short time.

On the other hand, the inventors of the present invention have found that in a case where, as described in JP2006-218442A, the magnetic particles are magnetically collected on the inner surface of the side wall surface of the reaction cell, and then a washing solution, a reagent, or the like is dispensed in a state in which the magnetic field is released, and the magnetic particles are redispersed, the dispersibility of the magnetic particles may be reduced.

In a case where the dispersibility of the magnetic particles is reduced, for example, a washing effect by a washing solution is reduced or reactivity with a reagent is reduced, which leads to the occurrence of a measurement error.

The present disclosure has been made in consideration of the above circumstances, and an object of the present disclosure is to provide a magnetic collection unit that makes it possible to improve the dispersibility of the magnetic particles that have been subjected to magnetic collection, and an examination device that makes it possible to improve the dispersibility of the magnetic particles and to suppress the occurrence of a measurement error.

A magnetic collection unit according to the present disclosure is a magnetic collection unit which, during a washing treatment of separating a labeling substance bound to an examination target substance and a labeling substance not bound to the examination target substance in an examination device using magnetic particles in a solid phase in an antigen-antibody reaction, generates a magnetic field in an inside of a reaction cell accommodating a suspension containing the magnetic particles and magnetically collects the magnetic particles in the suspension on an inner wall surface of the reaction cell, the magnetic collection unit comprising:
a magnetic field generation unit that includes a magnet having a length equal to or longer than a distance from a liquid surface of the suspension in the reaction cell to a bottom surface of the reaction cell and generating a magnetic field across a range from the liquid surface to the bottom surface; and
a moving mechanism that moves the magnetic field generation unit in a depth direction from the liquid surface toward the bottom surface in a state in which an upper end of the magnet is positioned at the liquid surface or above the liquid surface.

It is preferable that the magnetic field generation unit is disposed in a state in which a magnetic pole of the magnet is allowed to abut on a side surface of the reaction cell, and the moving mechanism moves the magnetic pole of the magnet in the depth direction in a state where the magnetic pole of the magnet is allowed to abut on the side surface of the reaction cell.

It is preferable that the magnet is a neodymium magnet.

The magnet may be an electromagnet.

It is preferable that the magnetic field generation unit includes two magnets having the length and a non-magnetic body, and the two magnets are disposed such that surfaces not having a magnetic pole face each other with the non-magnetic body being sandwiched therebetween, and it is preferable that the two magnets are disposed such that magnetic poles different from each other face the reaction cell.

The magnetic field generation unit may include a shield plate that blocks a magnetic force, at an end part of the two magnets in an arrangement direction.

A plurality of the magnetic field generation units may be disposed in parallel, and in this case, it is preferable that the moving mechanism integrally moves the plurality of the magnetic field generation units.

A shield plate that blocks a magnetic force may be provided in an arrangement direction at an end part of the plurality of the magnetic field generation units that are disposed in parallel.

An examination device according to the present disclosure comprises:
a washing treatment unit that includes the magnetic collection unit according to the present disclosure and carries out the washing treatment;
a detection unit that detects light due to the labeling substance; and
a transport mechanism that transports the reaction cell,
in which the washing treatment unit and the detection unit are disposed along a transport direction of the reaction cell.

According to the magnetic collection unit according to the technology of the present disclosure, it is possible to improve the dispersibility of the magnetic particles that have been subjected to magnetic collection. In addition, according to the examination device according to the technology of the present disclosure, it is possible to improve the dispersibility of the magnetic particles and to suppress the occurrence of a measurement error.

### BRIEF DESCRIPTION OF THE DRAWINGS

Fig. 1 is a schematic view showing an overall configuration of an examination device.
Fig. 2 is a view showing a treatment step in each unit in a treatment unit of the examination device.
Fig. 3 is a view showing a treatment step in each unit in the treatment unit of the examination device.
Fig. 4 is a view showing a main part of a washing treatment unit.
Fig. 5 is a view showing a relationship between a magnetic field generation unit of a magnetic collection unit and a reaction cell.
Fig. 6A is a view taken in a direction of an arrow VIA in Fig. 5, and Fig. 6B is a view taken in a direction of an arrow VIB in Fig. 5.
Fig. 7 is a view showing a washing treatment step (step ST11 to step ST15).
Fig. 8 is a view showing the washing treatment step (step ST16 to step ST21).
Fig. 9 is a perspective view showing a modification example of the magnetic field generation unit.
Fig. 10A is a view taken in a direction of an arrow XA in Fig. 9, and Fig. 10B is a view taken in a direction of an arrow XB in Fig. 9.
Fig. 11A is a top view of the reaction cell in a case where linear magnetic collection is carried out with a magnetic field generation unit, and Fig. 11B is a top view of the reaction cell in a case where linear magnetic collection is carried out with a magnetic field generation unit in the modification example.
Fig. 12A is a view showing a case where point magnetic collection is carried out with a magnetic field generation unit, and Fig. 12B is a view showing a case where point magnetic collection is carried out with a magnetic field generation unit in the modification example.
Fig. 13 is a view for describing an effect obtained by including a shield plate.
Fig. 14 is a view showing a washing treatment unit including a magnetic collection unit in which a plurality of magnetic field generation units are juxtaposed in parallel.
Fig. 15 is a view showing a configuration in which a shield plate is provided in a magnetic collection unit in which a plurality of magnetic field generation units are arranged.

### DESCRIPTION OF THE PREFERRED EMBODIMENTS

Hereinafter, an examination device according to an embodiment of the present disclosure will be described with reference to the drawings. In each drawing, the same constitutional elements are represented by the same reference numerals.

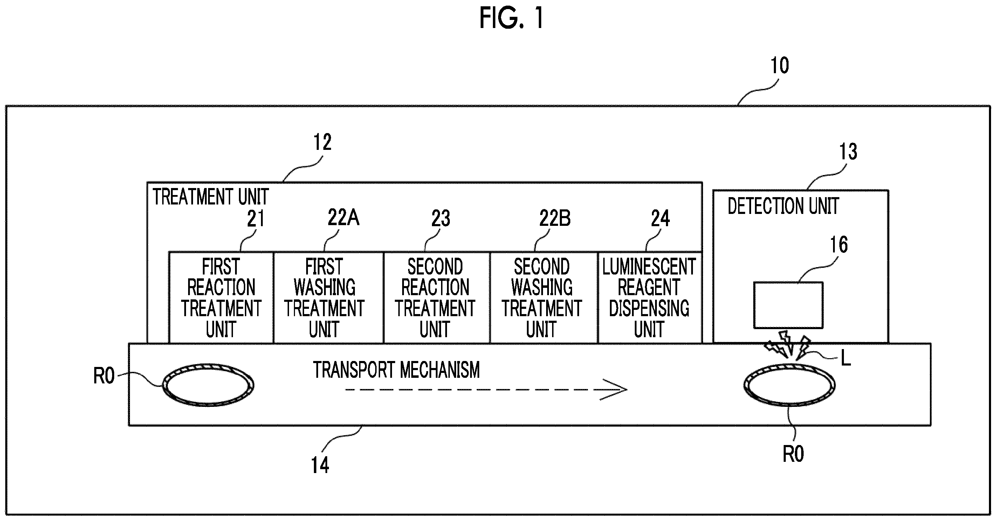

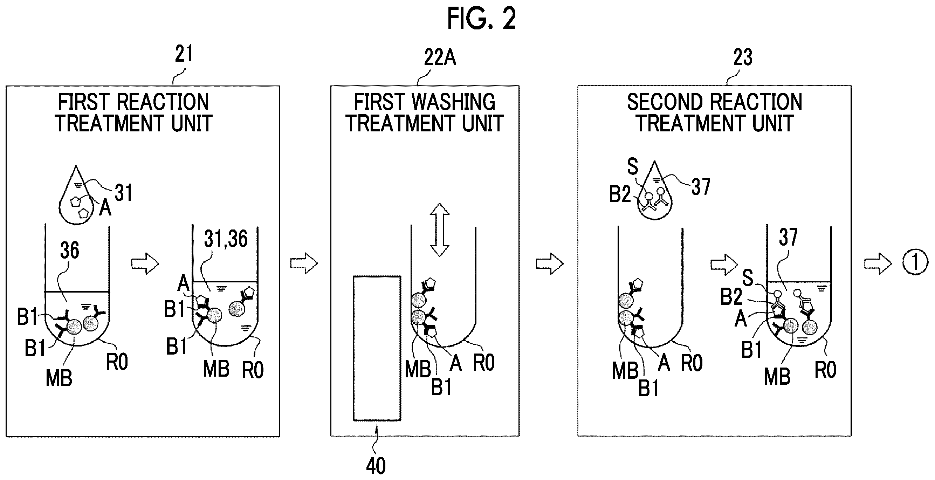

Fig. 1 is a schematic view showing an overall configuration of an examination device 10 according to the embodiment of the present disclosure. The examination device 10 is an immunological analysis device that detects an examination target substance by attaching a labeling substance to an examination target substance in a specimen by using an antigen-antibody reaction, and detecting light due to the labeling substance. The examination device 10 carries out an examination based on, for example, a chemiluminescent enzyme immunoassay method. The examination device 10 uses magnetic particles MB (see Fig. 2) as a solid phase of the antigen-antibody reaction. In the examination device 10, a reaction cell R0 containing the magnetic particles MB as a solid phase is used, and in the reaction cell R0, a treatment of attaching a labeling substance to an examination target substance in a specimen by an antigen-antibody reaction is carried out.

The specimen is, for example, a body fluid such as blood collected from the living body. In a case where the specimen is blood, the specimen may be any of whole blood, blood plasma, serum, or the like. In addition, the examination target substance that can be included in the specimen is an antigen, an antibody, a protein, and a low-molecular-weight compound. It is noted that the specimen is not limited to blood, and may be a substance collected from a living body, such as urine or body fluid.

In a case where the magnetic particles MB that are used as the solid phase have, for example, a spherical shape, the diameter thereof is, for example, 0.1 to 10 µm, preferably 0.1 to 5 µm, and more preferably about 1 to 3 µm. A first binding substance that specifically binds to an examination target substance is attached to the magnetic particles MB. As shown in Fig. 1, the examination device 10 includes, for example, a treatment unit 12, a detection unit 13, and a transport mechanism 14.

The transport mechanism 14 transports the reaction cell R0 in the examination device 10. The treatment unit 12 and the detection unit 13 are disposed along the transport direction of the reaction cell R0 that is transported by the transport mechanism 14. As a result, the reaction cell R0 transported by the transport mechanism 14 is sequentially transported to the treatment unit 12 and the detection unit 13.

In the detection unit 13, a detection treatment of detecting an examination target substance in the specimen is carried out. The detection unit 13 includes a photodetector 16 such as a photomultiplier tube or a photodiode. The photodetector 16 is disposed to face the reaction cell R0 and detects light L due to the labeling substance bound to the examination target substance. In the present example, an enzyme is used as the labeling substance, and chemiluminescence (hereinafter, referred to as chemiluminescence L) generated by a reaction between the enzyme and a luminescent substrate is detected as the light L due to the labeling substance. The photodetector 16 optically detects an examination target substance to which a labeling substance has been attached by receiving the chemiluminescence L. It is noted that the examination device 10 includes a processor that is not shown in the drawing, and the photodetector 16 outputs a light-receiving signal corresponding to the amount of light received, to the processor. The processor detects whether or not the specimen contains the examination target substance and the concentration thereof based on the light-receiving signal output from the photodetector 16.

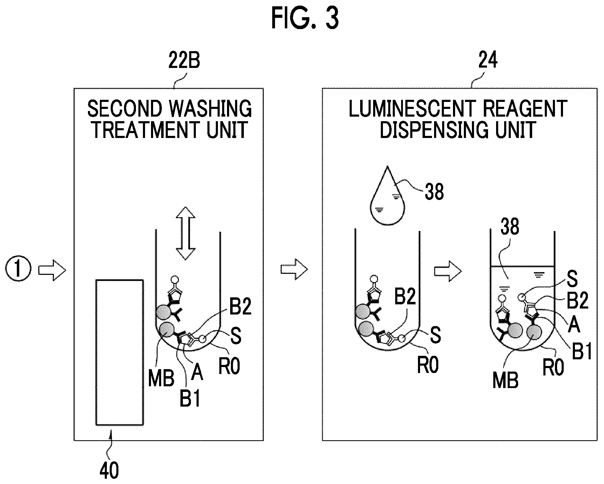

In the treatment unit 12, the treatment of attaching a labeling substance to the examination target substance is carried out by the antigen-antibody reaction described above. In the treatment unit 12, a first reaction treatment unit 21, a first washing treatment unit 22A, a second reaction treatment unit 23, a second washing treatment unit 22B, and a luminescent reagent dispensing unit 24 are disposed in this order from the upstream side in the transport direction along the transport direction of the reaction cell R0. Fig. 2 and Fig. 3 show views schematically showing a treatment that is carried out in each unit of the treatment unit 12.

As shown in Fig. 2, in the first reaction treatment unit 21, a specimen 31 is dispensed into the reaction cell R0, and the specimen 31 is mixed with a reagent 36 containing the magnetic particles MB to which a first binding substance B1 is fixed in the reaction cell R0. The first binding substance B1 is a substance that specifically binds to an examination target substance A, and in a case where the examination target substance A is present in the specimen 31, a first reaction in which the examination target substance A binds to the first binding substance B1 occurs. By this first reaction, an immune complex of the examination target substance A and the first binding substance B1 is formed, and the examination target substance A is captured by the magnetic particles MB through the first binding substance B1. It is noted that the first reaction is promoted by sufficiently dispersing the magnetic particles MB in the reagent 36 and the specimen 31.

As shown in Fig. 2, in the first washing treatment unit 22A, a washing treatment of carrying out B/F separation in which a reacted substance and an unreacted substance in a mixed liquid obtained from the reagent 36 containing the magnetic particles MB and the specimen 31 are separated is carried out. The first washing treatment unit 22A includes a magnetic collection unit 40 described later, and the magnetic collection unit 40 is used during the B/F separation. In the drawing, a bidirectional arrow schematically shows a state in which a liquid is taken in or taken out from the reaction cell R0. Details of the washing treatment will be described later.

As shown in Fig. 2, in the second reaction treatment unit 23, a labeling reagent 37 is dispensed into the reaction cell R0, and in the reaction cell R0, the labeling reagent 37 containing the second binding substance B2 to which a labeling substance S has been attached is mixed with the magnetic particles MB. The second binding substance B2 is a substance that specifically binds to the examination target substance A, and in a case where the examination target substance A is captured by the magnetic particles MB, a second reaction in which the second binding substance B2 binds to the examination target substance A occurs. By this second reaction, a sandwich-type immune complex in which the examination target substance A is sandwiched between the first binding substance B1 and the second binding substance B2 is formed, and the second binding substance B2 is captured by the magnetic particles MB through the examination target substance A and the first binding substance B1. It is noted that the second reaction is promoted by sufficiently dispersing the magnetic particles MB in the labeling reagent 37.

As shown in Fig. 3, in the second washing treatment unit 22B, a washing treatment of carrying out B/F separation in which a reacted substance and an unreacted substance in a mixed liquid of the magnetic particles MB and the labeling reagent 37 are separated is carried out. Similar to the first washing treatment unit 22A, the second washing treatment unit 22B includes the magnetic collection unit 40, and the magnetic collection unit 40 is used during the B/F separation. The washing treatment method is the same as the washing treatment in the treatment carried out by the first washing treatment unit 22A, and the details thereof will be described later. Even in Fig. 3 similar to in Fig. 2, a bidirectional arrow schematically shows a state in which a liquid is taken in or taken out from the reaction cell R0.

In the luminescent reagent dispensing unit 24, a luminescent reagent 38 is dispensed to the reaction cell R0, and the magnetic particles MB and the luminescent reagent 38 are mixed. The luminescent reagent 38 is a reagent that reacts with the labeling substance S to generate the chemiluminescence L (see Fig. 1). It is noted that the generation of the chemical luminescence L is promoted by sufficiently dispersing the magnetic particles MB in the luminescent reagent 38.

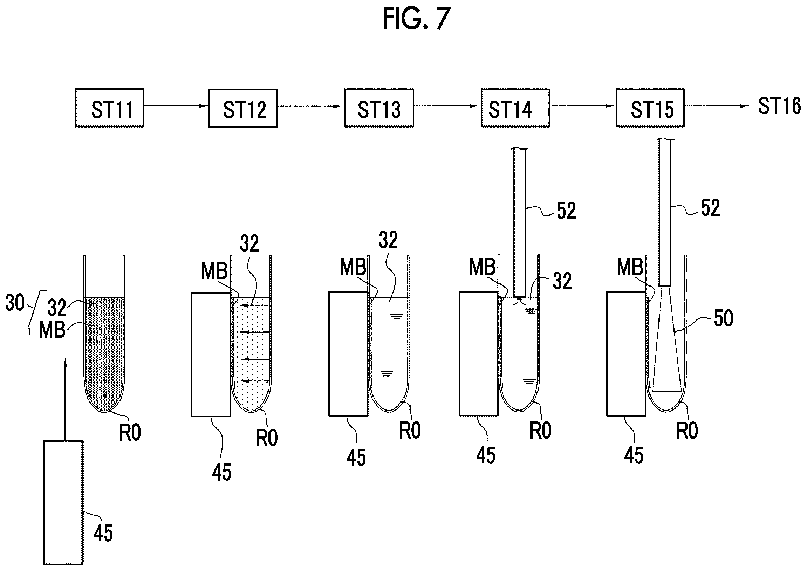

The first washing treatment unit 22A and the second washing treatment unit 22B have substantially the same configuration. Therefore, in a case where they are not particularly required to be distinguished from each other, the configuration and the function thereof will be described as the washing treatment unit 22. All of the mixed liquid of the reagent 36 containing the magnetic particles MB and the specimen 31, the mixed liquid of the magnetic particles MB and the labeling reagent 37, and the mixed liquid of the magnetic particles MB and the luminescent reagent 38 are a suspension 30 (see Fig. 7) in which the magnetic particles MB are dispersed in the liquid 32, and in the following description of the washing treatment, the mixed liquids described above are collectively referred to as the suspension 30. In addition, the liquid 32 is a general term for a reagent or the like in which the magnetic particles MB are dispersed.

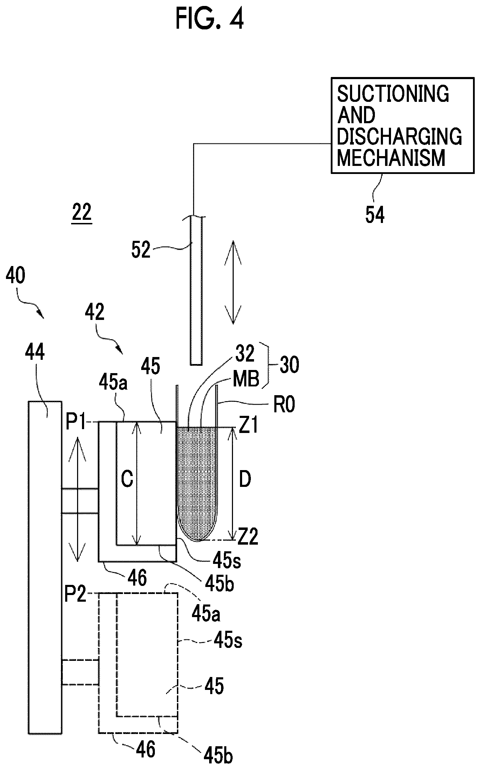

As shown in Fig. 4 as an example, the washing treatment unit 22 includes a suctioning and discharging mechanism 54 that includes the magnetic collection unit 40 that magnetically collects the magnetic particles MB in the reaction cell R0, and a nozzle 52 for suctioning and discharging the washing solution 50 to the reaction cell R0, and a moving mechanism (not shown in the drawing) of the nozzle 52.

The magnetic collection unit 40 generates a magnetic field in the inside of the reaction cell R0 and collects the magnetic particles MB in the suspension 30 on the inner wall surface of the reaction cell R0. The magnetic collection unit 40 includes a magnetic field generation unit 42 and a moving mechanism 44.

The magnetic field generation unit 42 includes a magnet 45. The magnet 45 has a length C equal to or longer than a distance D from at least a liquid surface Z1 of the suspension 30 in the reaction cell R0 to a bottom surface Z2 of the reaction cell R0. It is noted that the amount of the liquid 32 dispensed into the reaction cell R0 is determined in advance, and the position of the liquid surface Z1 is already known. As a result, the distance D is already known. The length C of the magnet 45 may be appropriately set according to the distance D. The length C of the magnet 45 needs only to be equal to or larger than the distance D; however, as shown in Fig. 4 as an example, it is desirable that the length C is larger than the distance D. It is noted that the length C of the magnet 45 is preferably a length equal to or less than 130% of the distance D.

In a case of collecting the magnetic particles MB in the suspension 30, the magnet 45 is disposed such that the magnet 45 is positioned in a range from the liquid surface Z1 to the bottom surface Z2 of the suspension 30 with the length direction of the magnet 45 being along the depth direction in the reaction cell R0. That is, during magnetic collection, the magnet 45 is disposed such that an upper end 45a of the magnet 45 is positioned at the position of the liquid surface Z1 or positioned above the liquid surface Z1, and a lower end 45b of the magnet 45 is positioned at the position of the bottom surface Z2 or below the bottom surface Z2. In a case where the magnet 45 is disposed in this way, the magnet 45 can generate a magnetic field at the same time in a range from the liquid surface Z1 to the bottom surface Z2 in the reaction cell R0.

It is noted that it is preferable that, during magnetic collection, the upper end 45a of the magnet 45 is positioned above the liquid surface Z1 by, for example, about 1 mm.

The magnet 45 is a permanent magnet or an electromagnet. In the present example, an example in which the magnet 45 is a permanent magnet is shown. As the permanent magnet, a neodymium magnet having a large magnetic force is particularly preferable.

The moving mechanism 44 moves the magnetic field generation unit 42 in a depth direction (vertical direction in the drawing) from the liquid surface Z1 toward the bottom surface Z2 in a state in which the upper end 45a of the magnet 45 is positioned at the position of the liquid surface Z1 or above the liquid surface Z1. In the present example, the moving mechanism 44 makes the magnet 45 movable between a first position where the upper end 45a of the magnet 45 is at a position P1 above the liquid surface Z1 and a second position where the upper end 45a of the magnet 45 is at a position P2 below the bottom surface Z2 of the reaction cell R0. The second position is a retreat position where the magnetic field from the magnet 45 has little effect on the inside of the reaction cell R0.

The magnet 45 is supported by a support unit 46, and the moving mechanism 44 moves the magnet 45 together with the support unit 46. The moving mechanism 44 is composed of, for example, a linear actuator and the like.

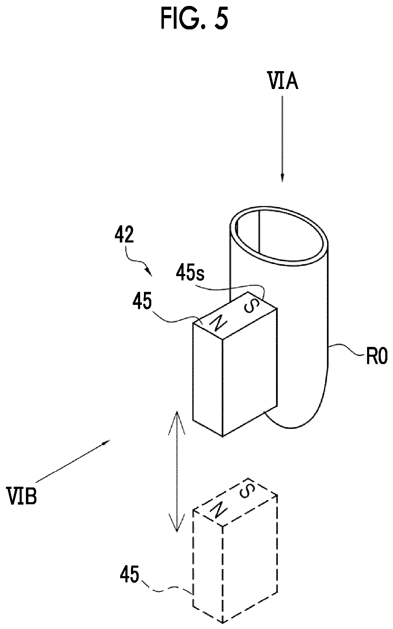

Fig. 5 is a perspective view showing a positional relationship between the magnet 45 of the magnetic field generation unit 42 and the reaction cell R0. As shown in Fig. 5, the magnet 45 is disposed such that a magnetic pole (here, the S pole) 45s abuts on the side surface of the reaction cell R0. In addition, the moving mechanism 44 is configured to be movable in the vertical direction, that is, in the depth direction from the liquid surface Z1 toward the bottom surface Z2 in a state in which the magnetic pole 45s is allowed to abut on the side surface of the reaction cell R0.

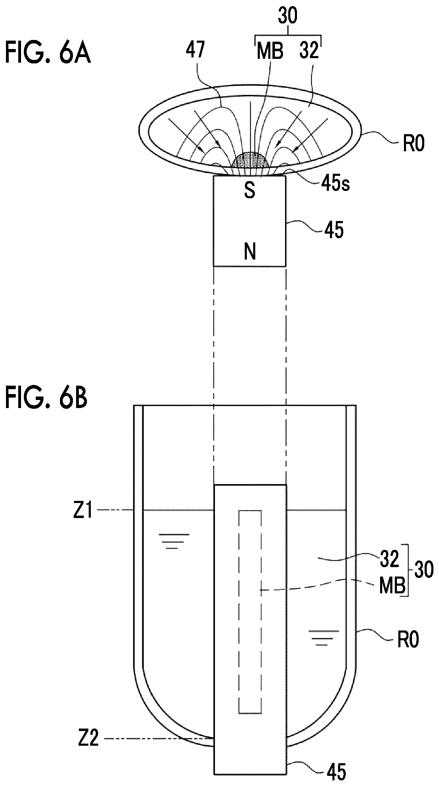

Fig. 6A is a view of the reaction cell R0 and the magnet 45 in Fig. 5, which is taken in a direction of an arrow VIA as viewed in the direction of the VIA, and Fig. 6B is a view of the reaction cell R0 and the magnet 45, which is taken in a direction of an arrow VIB as viewed in the direction of the VIB.

As shown in Fig. 6A, a magnetic field indicated by a magnetic force line 47 is generated in the reaction cell R0 by the magnet 45. The magnetic particles MB in the suspension 30 in the reaction cell R0 are attracted to the magnet 45, moved in the arrow direction, and magnetically collected on the inner wall surface of the reaction cell R0. As shown in Fig. 6B, the magnet 45 is disposed from the liquid surface Z1 to the bottom surface Z2 of the reaction cell R0. Therefore, the magnetic particles MB are magnetically collected in a linear region indicated by a broken line in the drawing along a length direction of the magnet 45.

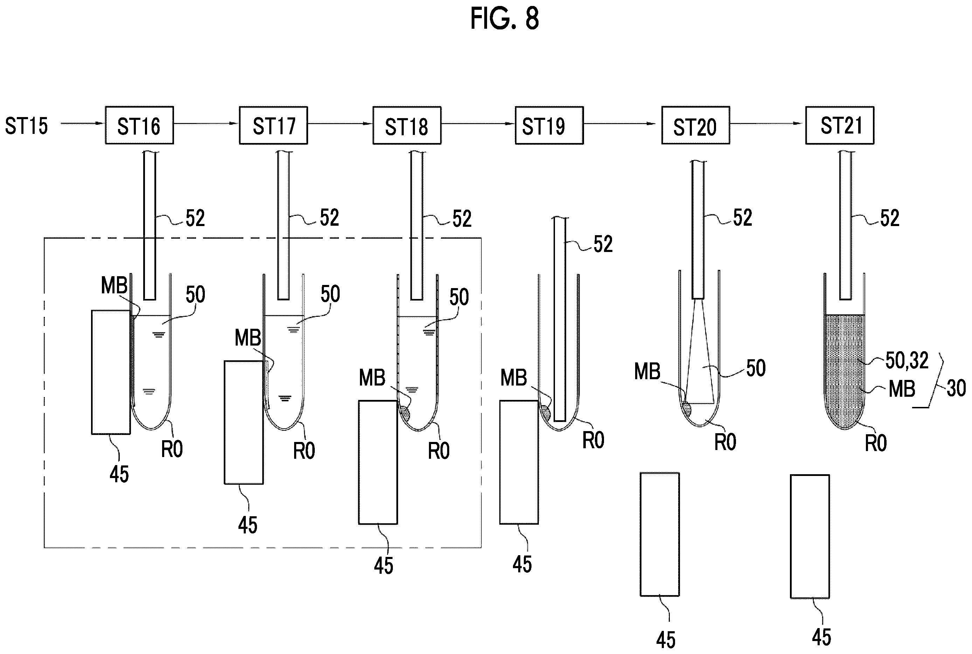

Here, the details of the washing treatment step of carrying out the B/F separation will be described with reference to Fig. 7 and Fig. 8.

In the reaction cell R0 immediately before the start of the washing treatment shown in the step ST11 of Fig. 7, the suspension 30 in which the magnetic particles MB are dispersed in the liquid 32 is accommodated. At this point in time, the magnet 45 is positioned at a position where the magnetic field is not applied to the reaction cell R0. From this state, the magnet 45 is moved upward and is disposed on the side surface of the reaction cell R0 as shown in the step ST12. As a result, the magnetic particles MB in the reaction cell R0 are attracted to the magnet 45 and moved in the arrow direction. Since the magnet 45 is disposed along the side wall surface of the reaction cell R0 from the liquid surface Z1 of the suspension 30 to the bottom surface Z2, the magnetic particles MB dispersed in the liquid 32 move substantially horizontally so that the movement is in the shortest distance toward the magnet 45 in the liquid 32.

Therefore, as shown in the step ST13, the magnetic particles MB are magnetically collected in a linear shape along the length direction of the magnet 45 on the inner wall surface of the reaction cell R0.

Next, as shown in the step ST14, a nozzle 52 for washing is inserted into the reaction cell R0 in a state in which the magnetic particles MB are magnetically collected in a linear shape on the inner wall surface of the reaction cell R0, and the liquid 32 in the reaction cell R0 is suctioned. The nozzle 52 for washing is gradually lowered to the bottom surface side of the reaction cell R0 while suctioning the liquid 32. Thereafter, as shown in the step ST15, the washing solution 50 is discharged from the nozzle 52 for washing in a state in which the nozzle 52 for washing is pulled up. It is noted that the step ST14 and the step ST15 of carrying out suction and discharge in a state in which the magnetic particles MB are magnetically collected may be repeated a plurality of times.

Thereafter, as shown in the step ST16 to the step ST18 of Fig. 8, the magnet 45 is gradually allowed to move downward along the wall surface of the reaction cell R0 in a state in which the magnetic particles MB are magnetically collected in a linear shape on the inner wall surface of the reaction cell R0. As the magnet 45 moves, the magnetic particles MB move downward, and the state of the magnetic particles MB is shifted from a state in which the magnetic particles MB are magnetically collected in a linear shape (see the step ST16) to a state in which the magnetic particles MB are magnetically collected in a dot shape on the inner wall surface close to the bottom surface of the reaction cell R0 (see the step ST18).

As shown in the step ST18, the movement of the magnet 45 is stopped in a state in which the magnetic particles MB are magnetically collected in a dot shape, and then, as shown in the step ST19, the washing solution 50 in the reaction cell R0 is suctioned by the nozzle 52 for washing. In this case, the magnetic particles MB are magnetically collected in a dot shape at a position deviated from the distal end of the nozzle 52 so that the nozzle 52 does not suction the magnetic particles MB.

After the washing solution 50 in the reaction cell R0 is suctioned, the magnet 45 is moved to the retracting position as shown in the step ST20. As a result, the state becomes such that the magnetic field generated by the magnet 45 does not affect the inside of the reaction cell R0. Thereafter, the washing solution 50 is discharged from the nozzle 52 into the reaction cell R0. As a result, the magnetic particles MB are dispersed in the washing solution 50 as shown in the step ST21. It is noted that in the step ST21 of Fig. 8, reference numerals 50 and 32 are written together to indicate that, in the present step, the washing solution 50 is the liquid 32 in which the magnetic particles MB are dispersed. As described above, the liquid 32 is a general term for a reagent or the like in which the magnetic particles MB are dispersed, and in the step ST21 of Fig. 8, the washing solution 50 corresponds to the liquid 32.

It is noted that in the washing treatment unit 22, the steps of the step ST11 to the step ST21 described above are repeated a plurality of times, for example, about three times. The B/F separation is carried out by this washing treatment step.

As described above, the magnetic collection unit 40 includes the magnetic field generation unit 42 including the magnet 45 which has a length C equal to or longer than a distance D from a liquid surface Z1 of the suspension 30 in the reaction cell R0 to a bottom surface Z2 of the reaction cell R0 and generates a magnetic field across a range from the liquid surface Z1 to the bottom surface Z2. Therefore, in a case where the magnet 45 is disposed such that the magnet 45 is positioned in a range from the liquid surface Z1 to the bottom surface Z2 of the suspension 30 with the length direction of the magnet 45 being along the depth direction in the reaction cell R0, the magnet 45 can generate a magnetic field at the same time in a range from the liquid surface Z1 to the bottom surface Z2 in the reaction cell R0. In a case where a magnet having a length shorter than the distance D between the liquid surface Z1 and the bottom surface Z2 is used, at least a part of the magnetic particles MB in the suspension 30 move in an oblique direction intersecting the horizontal direction, thereby reaching the inner wall surface. On the other hand, in the present embodiment, since substantially all of the magnetic particles MB in the suspension 30 move in a substantially horizontal direction and are magnetically collected in the reaction cell R0, the magnetic particles MB reach the inner wall surface in the shortest distance. As a result, the magnetic collection of the magnetic particles MB can be carried out very quickly.

In addition, the magnetic collection unit 40 includes the moving mechanism 44 and moves the magnetic field generation unit 42 (here, the magnet 45) in a depth direction from the liquid surface Z1 toward the bottom surface Z2 in a state in which the upper end 45a of the magnet 45 is positioned at the liquid surface Z1 or above the liquid surface Z1. As a result, the magnetic particles MB that have been magnetically collected in a linear shape in the length direction of the magnet 45 can be magnetically collected in a dot shape in the vicinity of the bottom surface of the reaction cell R0. In a case where the magnetic particles MB are redispersed in the liquid by changing the magnetic collection form from a linear shape to a dot shape in this way, suctioning the liquid 32, and then discharging the washing solution 50 from the nozzle 52 such that the washing solution 50 comes into contact with the magnetic particles MB that have been magnetically collected in a dot shape, the dispersibility of the magnetic particles MB can be improved. In a case where the dispersibility of the magnetic particles MB is increased, the washability of the magnetic particles MB is improved, and the B/F separation can be carried out with higher accuracy. In the examination device 10, by using such a magnetic collection unit 40, it is possible to reduce noise associated with the effect of improving the B/F separation accuracy, and, in the long run, it is possible to suppress the occurrence of a measurement error and obtain a highly accurate measurement result.

In the magnetic collection unit 40 shown in Fig. 4, the moving mechanism 44 is configured to collect the magnetic particles MB in a linear shape, change the magnetic collection form to a dot shape, and further, be movable only in the uniaxial direction (vertical direction) to the retreat position. However, the form of the moving mechanism 44 is not limited thereto, and the moving mechanism 44 may be configured to be movable in the vertical direction in a range in which the magnetic collection form of the magnetic particles MB can be changed from a linear shape to a dot shape, and further, may be configured to be movable in a direction away from the reaction cell R0 in the horizontal direction. In this case, the retreat position may be such a position away from the reaction cell R0 in the horizontal direction that the magnetic field from the magnetic field generation unit 42 does not affect the reaction cell R0.

In the magnetic collection unit 40 described above, the magnetic field generation unit 42 is disposed in a state in which the magnetic pole 45s of the magnet 45 is allowed to abut on a side surface of the reaction cell R0, and the moving mechanism 44 moves the magnetic field generation unit 42 in the depth direction in a state where the magnetic pole 45s of the magnet 45 is allowed to abut on the side surface of the reaction cell R0. The magnetic field generation unit 42 may be disposed such that the magnetic pole 45s of the magnet 45 is brought close to the side surface of the reaction cell R0 without abutting on the side surface. However, by causing the magnetic pole 45s to abut on the reaction cell R0, the intensity of the magnetic field generated in the reaction cell R0 can be increased, the magnetic collection effect can be increased, and the magnetic collection can be carried out more quickly. In addition, even in a case where the magnetic field generation unit 42 is moved, the magnetic field generation unit 42 is moved in a state in which the magnetic pole 45s is allowed to abut on the side surface of the reaction cell R0, whereby the magnetic particles MB smoothly follow the magnet 45 in a case where the magnetic collection form is changed from a linear shape to a dot shape.

By using a strong magnet such as a neodymium magnet as the magnet 45, it is possible to reliably generate a magnetic field in the reaction cell R0 and enhance the magnetic collection function of the magnetic particles MB. The magnet provided in the magnetic field generation unit 42 is not limited to a permanent magnet, and an electromagnet may be used. In a case where an electromagnet is used, the on and off of the magnetic field generated in the reaction cell R0 can be switched by the on and off of the current, Therefore, it is not necessary to move the magnet to the retreat position in a case where the current is turned off after the magnet is moved to change the magnetic collection form from a linear shape to a dot shape and the liquid is suctioned. On the other hand, in a case where a permanent magnet is used, electric power for generating a magnetic field is not required, and it is possible to simplify a wiring line and the like.

In the above-described embodiment, as shown in Fig. 5, an example in which the magnetic field generation unit 42 includes only one magnet 45 and one magnetic pole 45s thereof is disposed to face the side surface of the reaction cell R0, thereby carrying out magnetic collection has been described. The magnetic collection unit according to the present disclosure is not limited to a form in which only one magnet 45 is provided.

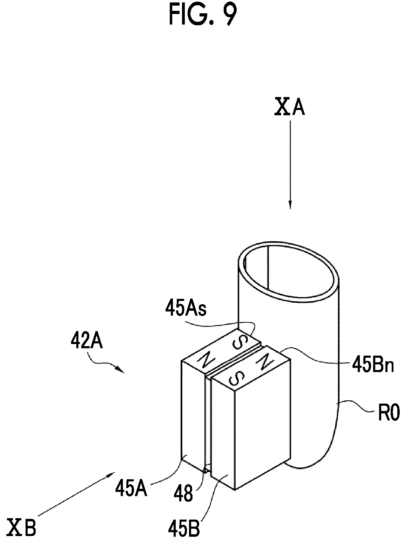

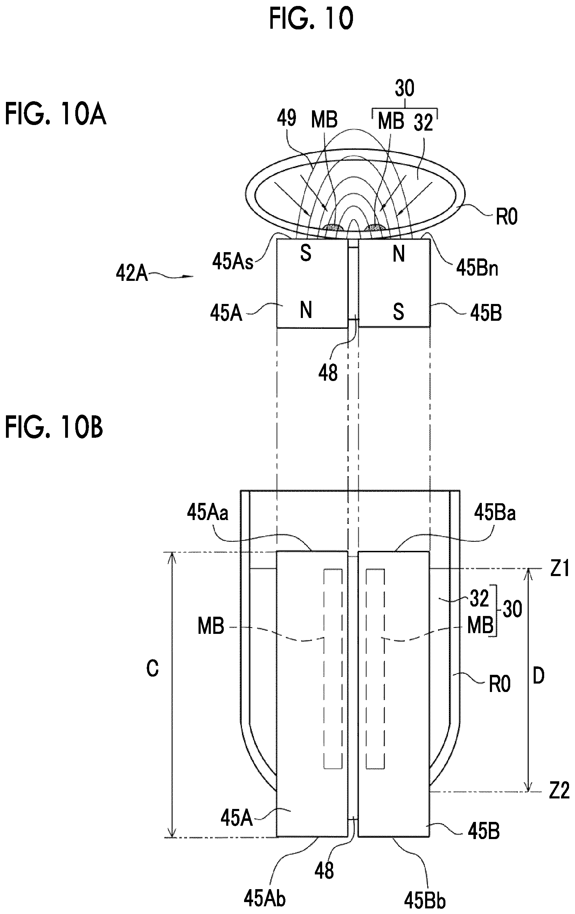

Fig. 9 shows a magnetic field generation unit 42Ain a modification example. Fig. 10A is a view of the reaction cell R0 and the magnetic field generation unit 42A in Fig. 9, which is taken in a direction of an arrow XA as viewed in the direction of the XA, and Fig. 10B is a view of the reaction cell R0 and the magnetic field generation unit 42A, which is taken in a direction of an arrow XB as viewed in the direction of the XB.

The magnetic field generation unit 42A in the modification example includes two magnets 45A and 45B and a non-magnetic body 48 sandwiched between the two magnets 45A and 45B. The two magnets 45A and 45B are disposed such that surfaces having no magnetic poles face each other with the non-magnetic body 48 being sandwiched therebetween. In addition, the two magnets 45A and 45B are disposed such that magnetic poles different from each other face the reaction cell R0. In the example of Fig. 9, the magnet 45A is disposed such that the magnetic pole 45As which is an S pole faces the side surface of the reaction cell R0, and the magnet 45B is disposed such that the magnetic pole 45Bn which is an N pole faces the side surface of the reaction cell R0.

As shown in Fig. 10B, similar to the magnet 45, the magnets 45A and 45B in the present example have a length C equal to or longer than a distance D from at least a liquid surface Z1 of the suspension 30 in the reaction cell R0 to a bottom surface Z2 of the reaction cell R0. In addition, during magnetic collection, the magnets 45A and 45B are disposed such that the magnets 45A and 45B are positioned in a range from the liquid surface Z1 to the bottom surface Z2 of the suspension 30 with the length directions of the magnets 45A and 45B being along the depth direction in the reaction cell R0. In the present example, the upper end 45Aa of the magnet 45A and the upper end 45Ba of the magnet 45B are each positioned above the liquid surface Z1. In addition, a lower end 45Ab of the magnet 45A and a lower end 45Bb of the magnet 45B are positioned below the bottom surface Z2.

It is suitable that the non-magnetic body 48 is, for example, an aluminum plate consisting of aluminum, which has a flat plate shape as an example.

As shown in Fig. 10A, a magnetic field indicated by a magnetic force line 49 is generated in the reaction cell R0 by the magnetic field generation unit 42A. The magnetic particles MB in the suspension 30 in the reaction cell R0 are attracted to the magnets 45A and 45B, moved horizontally in the arrow direction, and magnetically collected on the inner wall surface of the reaction cell R0. In this case, the magnetic particles MB are magnetically collected in a linear shape along each of the magnet 45A and the magnet 45B. That is, the magnetic particles MB are magnetically collected in a linear shape of two lines on the inner wall surface of the reaction cell R0.

Since the two magnets 45A and 45B are provided, and the non-magnetic body 48 is provided therebetween, the magnetic field intensity can be improved, and the magnetic collection force can be improved. As shown by the magnetic force line 49 in Fig. 10A, by causing the magnetic pole 45As and the magnetic pole 45Bn different from each other to be adjacent to each other with the non-magnetic body 48 being sandwiched therebetween, it is possible to form a portion in a linear shape of two lines, which has a high magnetic field intensity. Therefore, the magnetic particles can be magnetically collected in a linear shape of two lines. The magnetic particles MB can be magnetically collected quickly as compared with a case where only one magnet 45 is provided.

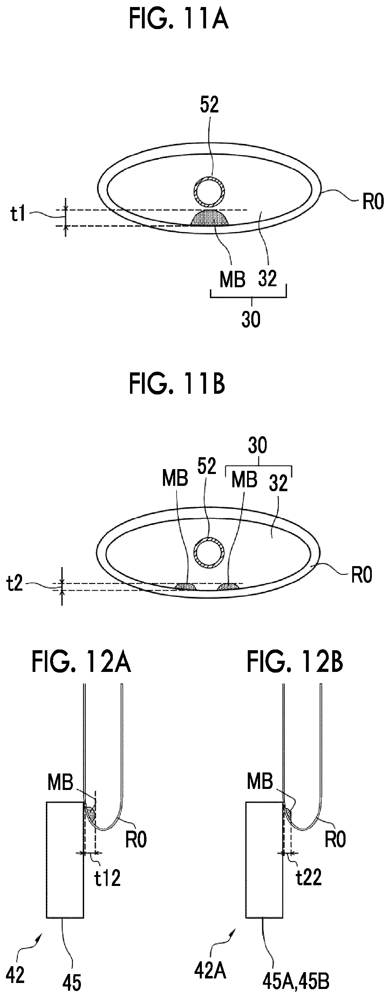

In addition, by magnetically collecting the magnetic particles MB in a linear shape of two lines, the thickness t2 (see Fig. 11B) of the aggregate of the magnetic particles MB that have been magnetically collected can be made smaller than that in a case where the magnetic particles MB are magnetically collected in a linear shape of one line (see Fig. 11A), and the loss of the magnetic particles MB during the suction of the liquid 32 by the nozzle 52 can be suppressed.

Fig. 11A shows a top view showing a state in which the nozzle 52 is inserted into the reaction cell R0 in which the magnetic particles MB are magnetically collected in a linear shape of one line shown in Fig. 6A. Fig. 11B is a top view showing a state in which the nozzle 52 is inserted into the reaction cell R0 in which the magnetic particles MB are magnetically collected in a linear shape of two lines shown in Fig. 10A. In Fig. 11A and Fig. 11B, the nozzle 52 is shown in a cross section.

The thickness t2 of the aggregate of the magnetic particles MB that have been magnetically collected in a linear shape of two lines in the reaction cell R0 shown in Fig. 11B is smaller than the thickness t1 of the aggregate of the magnetic particles MB that have been magnetically collected in a linear shape of one line in the reaction cell R0 shown in Fig. 11A. Here, the thicknesses t1 and t2 of the aggregate of the magnetic particles MB are distances between the inner wall surface corresponding to the side surface of the reaction cell R0 where the magnet 45 or the magnets 45A and 45B are allowed to abut on and the position of the aggregate of the magnetic particles MB closest to the central side of the reaction cell R0. In a case where the nozzle 52 is inserted into the reaction cell R0 in order to suction the liquid 32 in a state in which the magnetic particles MB are magnetically collected, the smaller the thickness of the aggregate of magnetic particles MB is, the more the distal end of the nozzle 52 can be separated from the magnetic particles MB.

As shown in Fig. 11A, in a case where the magnetic particles MB are magnetically collected in a linear shape of one line, the distance between the nozzle 52 and the aggregate of the magnetic particles MB is shortened. As the distance between the nozzle 52 and the aggregate of the magnetic particles MB becomes shorter, the distal end of the nozzle is more likely to come into contact with the magnetic particles MB during the insertion of the nozzle 52. On the other hand, as shown in Fig. 11B, in a case where the magnetic particles MB are magnetically collected in a linear shape of two lines, the nozzle 52 can be inserted into the reaction cell R0 in a state in which a certain distance is ensured from the aggregate of the magnetic particles MB. In a case where the magnetic particles MB come into contact with the distal end of the nozzle 52 and are attached to the distal end of the nozzle 52 during the insertion of the nozzle 52 and/or in a case where the distance from the distal end of the nozzle 52 to the aggregate of the magnetic particles MB is close during the suction, the magnetic particles MB are easily suctioned during the suction of the liquid 32 by the nozzle 52. As compared with a case where the magnetic particles MB shown in Fig. 11A are magnetically collected in a linear shape of one line, the thickness of the aggregate of the magnetic particles MB becomes smaller (t1 > t2) in a case where the magnetic particles MB shown in Fig. 11B are magnetically collected in a linear shape of two lines. Therefore, in a case where the magnetic particles MB are magnetically collected in a linear shape of two lines, it is possible to suppress the contact with the aggregate of the magnetic particles MB in a case where the nozzle 52 is inserted into the reaction cell R0. In addition, it is possible to further separate the distance between the distal end of the nozzle 52 and the aggregate of the magnetic particles MB during suction. As a result, the suction of the magnetic particles MB can be suppressed. It is noted that since the loss of the magnetic particles MB due to the suction of the magnetic particles MB leads to a decrease in measurement accuracy, the decrease in measurement accuracy can be suppressed by suppressing the suction of the magnetic particles MB.

Fig. 12A is a view in a case where the magnetic field generation unit 42 including only one magnet 45 changes the magnetic collection form from a linear shape to a dot shape, and Fig. 12B is a view in a case where the magnetic field generation unit 42Aincluding two magnets 45A and 45B changes the magnetic collection form from a linear shape to a dot shape. The thickness t22 of the aggregate of the magnetic particles MB from the inner wall surface of the reaction cell R0 in the case of Fig. 12B is smaller than the thickness t12 of the aggregate of the magnetic particles MB from the inner wall surface of the reaction cell R0 in the case of Fig. 12A. In the magnetic field generation unit 42A in the modification example, the magnetic collection is carried out in a linear shape of two lines by the two magnets 45A and 45B. Therefore, the magnetic collection is carried out in a shape of two dots in a case where the magnetic collection form is changed from a linear shape to a dot shape. Therefore, the thickness t22 of the aggregate magnetically collected in a shape of one dot can be reduced as compared with a case where one magnet 45 is used.

In a case where the nozzle 52 is inserted into the reaction cell R0, the smaller the thickness t22 of the aggregate of the magnetic particles MB is, the more the distal end of the nozzle 52 can be separated from the magnetic particles MB. Therefore, in a case where the magnetic collection is carried out by the magnetic field generation unit 42A, the effect of suppressing the particle loss caused by the suction of the magnetic particles MB by the nozzle 52 during the suction is high as compared with a case where the magnetic field is magnetically collected by the magnetic field generation unit 42.

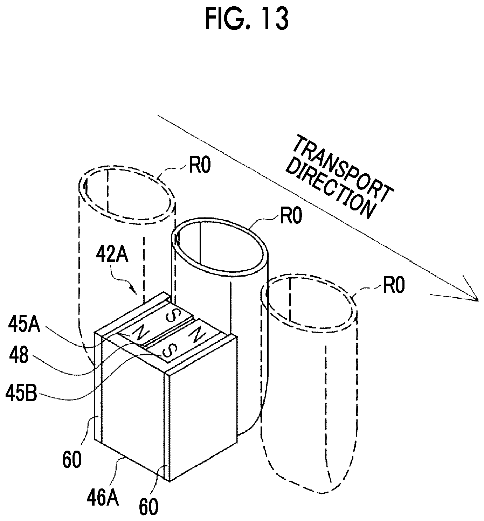

It is noted that the magnetic field generation unit 42A in the modification example is supported by a support unit 46A (see Fig. 13) and then is moved in the vertical direction by the moving mechanism 44 together with the support unit 46A, similar to the magnetic field generation unit 42 shown in Fig. 4.

In addition, as shown in Fig. 13, it is preferable that the magnetic field generation unit 42A includes a shield plate 60 that blocks a magnetic force, at an end part of the two magnets in an arrangement direction 45A and 45B. In the example shown in Fig. 13, the shield plate 60 is provided outside the support unit 46A.

In the examination device 10 (see Fig. 1), the reaction cell R0 is transported in a transport direction indicated by an arrow in Fig. 13 by the transport mechanism 14. In this case, the transport mechanism 14 can transport a plurality of reaction cells R0 at the same time, and as shown in Fig. 13, the plurality of reaction cells R0 are transported in a state of being adjacent to each other. As shown in Fig. 1, the respective treatment units are disposed in order along the transport direction. Therefore, in a case where the magnetic field generation unit 42A generates, for B/F separation, a magnetic field shown by a solid line in Fig. 13 with respect to the reaction cell R0, the reaction cells R0 disposed adjacent to each other, which are indicated by a broken line may be subjected to a treatment that is required to disperse the magnetic particles MB in the liquid. The treatment that is required to disperse the magnetic particles MB in the liquid is, for example, a first reaction treatment, a second reaction treatment, or a luminescent reagent dispensing treatment. In a case where the magnetic field generated by the magnetic field generation unit 42A affects the reaction cell R0 that has been subjected to the treatment required to disperse the magnetic particles MB in the liquid, it is also conceivable that the deviation may occur in the magnetic particles MB in the reaction cell R0, and the dispersibility may be lowered. On the other hand, by providing the shield plate 60, it is possible to suppress the generation of a magnetic field in the reaction cell R0 adjacent to the reaction cell R0 that is a target in which a magnetic field is generated. That is, by providing the shield plate 60, it is possible to reduce adverse effects such as inhibition of the dispersibility of the magnetic particles MB in the reaction cell R0 adjacent to the reaction cell R0 that is a target in which a magnetic field is generated.

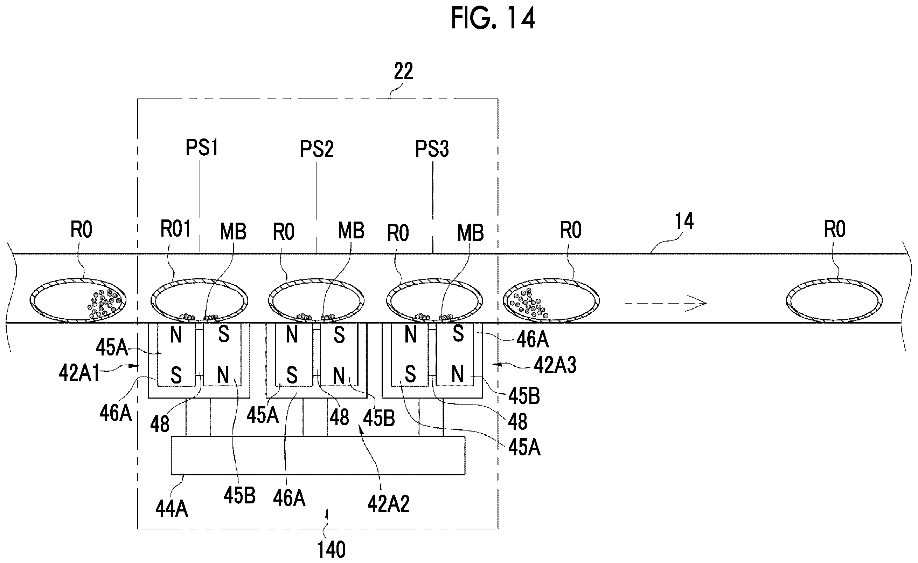

Further, in the magnetic collection unit according to the present disclosure, a plurality of the magnetic field generation units 42 (or 42A) may be disposed in parallel so that a magnetic field can be generated at the same time for the plurality of reaction cells R0. Fig. 14 shows a magnetic collection unit 140 including a plurality of magnetic field generation units 42A.

In the magnetic collection unit 140 shown in Fig. 14, three magnetic field generation units 42A are juxtaposed in parallel. In Fig. 14, for convenience, detailed reference numerals 1 to 3 are given for the reference numerals of the magnetic field generation units 42A1, 42A2, and 42A3, and the magnetic field generation unit 42A. In a case where they are not required to be distinguished from each other, they are simply referred to as the magnetic field generation unit 42A.

In the three magnetic field generation units 42A, magnets adjacent to each other between the magnetic field generation units 42A that are arranged adjacent to each other are arranged such that magnetic poles different from each other are adjacent to each other. For example, in Fig. 14, in the magnetic field generation unit 42A1 and the magnetic field generation unit 42A2 which are disposed adjacent to each other, the magnet 45B disposed on the magnetic field generation unit 42A2 side of the magnetic field generation unit 42A1 and the magnet 45A disposed on the magnetic field generation unit 42A1 side of the magnetic field generation unit 42A2 are disposed such that the S pole and the N pole are adjacent to each other.

The magnetic field generation units 42A are each supported by the support unit 46A, and the moving mechanism 44A is configured such that the three magnetic field generation units 42A are movable together with each of the support units 46A. The moving mechanism 44A integrally moves the three magnetic field generation units 42A. Similar to the moving mechanism 44 described above, the moving mechanism 44A is composed of, for example, a linear actuator and the like.

In the examination device 10 of Fig. 1, the transport mechanism 14 transports a plurality of reaction cells R0 at the same time, and the plurality of reaction cells R0 are subjected to a treatment in parallel in each treatment unit. As described above, the washing treatment step (step ST11 to Step ST21) described with reference to Fig. 7 and Fig. 8 is repeated, for example, three times. In this case, as shown in Fig. 14, three reaction cells R0 can be subjected to the washing treatment at the same time in a case where the washing treatment unit 22 is provided from the position PS1 to the position PS3, and the washing treatment step can be carried out at each of the position PS1, the position PS2, and the position PS3. In Fig. 14, for convenience of description, a detailed reference numeral 1 is given to one reaction cell R0 present at the position PS1 and is indicated as a reaction cell R01. For example, after subjecting the reaction cell R01 to the first washing treatment at the position PS1, the reaction cell R01 is moved to the position PS2 and subjected to the second washing treatment, and further, the reaction cell R01 is moved to the position PS3 and subjected to the third washing treatment. In this way, in a case where the washing treatment is carried out at each position while sequentially carrying out transporting from the position PS1 to the position PS3, the treatment can be efficiently carried out.

In a case where the magnetic collection unit 140 is used, three magnetic field generation units 42A can be integrally driven and magnetic collection can be carried out by one moving mechanism 44A in a case where three reaction cells R0 transported to three positions PS1 to PS3 are subjected to the washing treatment. In the magnetic collection unit 140, since the three magnetic field generation units 42A are integrally driven by one moving mechanism 44A, the cost can be suppressed as compared with a case where the moving mechanism is individually provided at each of the position PS1 to the position PS3.

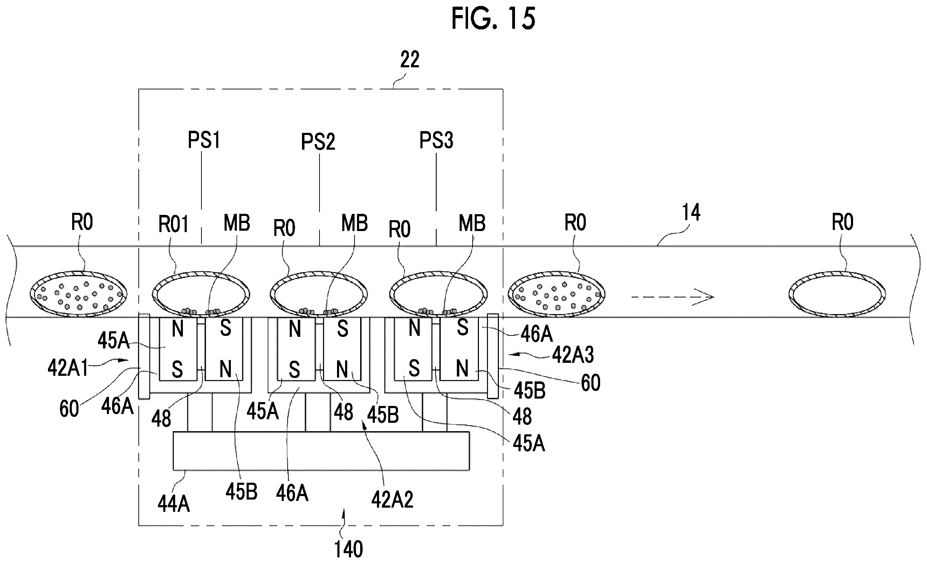

As shown in Fig. 15, it is preferable that the shield plate 60 that blocks a magnetic force in an arrangement direction at an end part of the plurality of the magnetic field generation units 42A that are disposed in parallel is provided in the magnetic collection unit 140 as well. As shown in Fig. 14, in a case where the shield plate 60 is not provided, the magnetic field generated by the magnetic field generation unit 42A may affect the reaction cell R0 positioned outside the washing treatment unit 22, which is disposed adjacent to the reaction cell R0 that is a target in which a magnetic field is generated. In this case, as shown in Fig. 14, the deviation occurs in the magnetic particles MB in the reaction cell R0 disposed adjacent to the reaction cell R0 that is a target in which a magnetic field is generated. On the other hand, as shown in Fig. 15, in a case where the shield plate 60 is provided, it is possible to suppress the influence of the magnetic field generated by the magnetic field generation unit 42A on the reaction cell R0 that is not a target in which a magnetic field is generated. That is, in a case where the shield plate 60 is provided, it is possible to suppress a decrease in the dispersibility of the magnetic particles MB in the liquid in the reaction cell R0 that is not a target in which a magnetic field is generated, that is, in the reaction cell R0 that is positioned outside the washing treatment unit 22.

In regard to the embodiment described above, the following supplementary notes will be further disclosed.

### (Supplementary Note 1)

A magnetic collection unit which, during a washing treatment of separating a labeling substance bound to an examination target substance and a labeling substance not bound to the examination target substance in an examination device using magnetic particles in a solid phase in an antigen-antibody reaction, generates a magnetic field in an inside of a reaction cell accommodating a suspension containing the magnetic particles and magnetically collects the magnetic particles in the suspension on an inner wall surface of the reaction cell, the magnetic collection unit comprising:
a magnetic field generation unit that includes a magnet having a length equal to or longer than a distance from a liquid surface of the suspension in the reaction cell to a bottom surface of the reaction cell and generating a magnetic field across a range from the liquid surface to the bottom surface; and
a moving mechanism that moves the magnetic field generation unit in a depth direction from the liquid surface toward the bottom surface in a state in which an upper end of the magnet is positioned at the liquid surface or above the liquid surface.

### (Supplementary Note 2)

The magnetic collection unit according to the supplementary note 1,
wherein the magnetic field generation unit is disposed in a state in which a magnetic pole of the magnet is allowed to abut on a side surface of the reaction cell, and
the moving mechanism moves the magnetic pole of the magnet in the depth direction in a state where the magnetic pole of the magnet is allowed to abut on the side surface of the reaction cell.

### (Supplementary Note 3)

The magnetic collection unit according to the supplementary note 1 or the supplementary note 2, wherein the magnet is a neodymium magnet.

### (Supplementary Note 4)

The magnetic collection unit according to the supplementary note 1 or the supplementary note 2, wherein the magnet is an electromagnet.

### (Supplementary Note 5)

The magnetic collection unit according to any one of the supplementary note 1 to the supplementary note 4, wherein the magnetic field generation unit includes two magnets having the length and a non-magnetic body, the two magnets are disposed such that surfaces not having a magnetic pole face each other with the non-magnetic body being sandwiched therebetween, and the two magnets are disposed such that magnetic poles different from each other face the reaction cell.

### (Supplementary Note 6)

The magnetic collection unit according to the supplementary note 5, wherein the magnetic field generation unit includes a shield plate that blocks a magnetic force, at an end part of the two magnets in an arrangement direction.

### (Supplementary Note 7)

The magnetic collection unit according to any one of the supplementary note 1 to the supplementary note 6,
wherein a plurality of the magnetic field generation units are disposed in parallel, and
the moving mechanism integrally moves the plurality of the magnetic field generation units.

### (Supplementary Note 8)

The magnetic collection unit according to the supplementary note 7, wherein a shield plate that blocks a magnetic force is provided in an arrangement direction at an end part of the plurality of the magnetic field generation units that are disposed in parallel.

### (Supplementary Note 9)

An examination device comprising:
a washing treatment unit that includes the magnetic collection unit according to any one of the supplementary note 1 to the supplementary note 8 and carries out the washing treatment;
a detection unit that detects light due to the labeling substance; and
a transport mechanism that transports the reaction cell,
wherein the washing treatment unit and the detection unit are disposed along a transport direction of the reaction cell.

It is noted that the disclosure of JP2022-175124 filed on October 31, 2022, is incorporated in the present specification in its entirety by reference. All of the documents, the patent applications, and the technical standards described in the present specification are incorporated into the present specification by reference to the same extent as in a case in which each of the documents, the patent applications, and the technical standards are specifically and individually stated to be described by reference.

## Claims

1. A magnetic collection unit which, during a washing treatment of separating a labeling substance bound to an examination target substance and a labeling substance not bound to the examination target substance in an examination device using magnetic particles in a solid phase in an antigen-antibody reaction, generates a magnetic field in an inside of a reaction cell accommodating a suspension containing the magnetic particles and magnetically collects the magnetic particles in the suspension on an inner wall surface of the reaction cell, the magnetic collection unit comprising:
a magnetic field generation unit that includes a magnet having a length equal to or longer than a distance from a liquid surface of the suspension in the reaction cell to a bottom surface of the reaction cell and generating a magnetic field across a range from the liquid surface to the bottom surface; and
a moving mechanism that moves the magnetic field generation unit in a depth direction from the liquid surface toward the bottom surface in a state in which an upper end of the magnet is positioned at the liquid surface or above the liquid surface.

2. The magnetic collection unit according to claim 1,
wherein the magnetic field generation unit is disposed in a state in which a magnetic pole of the magnet is allowed to abut on a side surface of the reaction cell, and
the moving mechanism moves the magnetic pole of the magnet in the depth direction in a state where the magnetic pole of the magnet is allowed to abut on the side surface of the reaction cell.

3. The magnetic collection unit according to claim 1, wherein the magnet is a neodymium magnet.

4. The magnetic collection unit according to claim 1, wherein the magnet is an electromagnet.

5. The magnetic collection unit according to any one of claims 1 to 4, wherein the magnetic field generation unit includes two magnets having the length and a non-magnetic body, the two magnets are disposed such that surfaces not having a magnetic pole face each other with the non-magnetic body being sandwiched therebetween, and the two magnets are disposed such that magnetic poles different from each other face the reaction cell.

6. The magnetic collection unit according to claim 5, wherein the magnetic field generation unit includes a shield plate that blocks a magnetic force, at an end part of the two magnets in an arrangement direction.

7. The magnetic collection unit according to any one of claims 1 to 4,
wherein a plurality of the magnetic field generation units are disposed in parallel, and
the moving mechanism integrally moves the plurality of the magnetic field generation units.

8. The magnetic collection unit according to claim 7, wherein a shield plate that blocks a magnetic force is provided in an arrangement direction at an end part of the plurality of the magnetic field generation units that are disposed in parallel.

9. An examination device comprising:
a washing treatment unit that includes the magnetic collection unit according to claim 1 and carries out the washing treatment;
a detection unit that detects light due to the labeling substance; and
a transport mechanism that transports the reaction cell,
wherein the washing treatment unit and the detection unit are disposed along a transport direction of the reaction cell.
